(19) Europäisches Patentamt
European Patent Office
Office européen des brevets

(11) **EP 2 950 829 B1**

(12) **EUROPÄISCHE PATENTSCHRIFT**

(45) Veröffentlichungstag und Bekanntmachung des Hinweises auf die Patenterteilung:
**11.03.2020  Patentblatt 2020/11**

(21) Anmeldenummer: **14700906.2**

(22) Anmeldetag: **20.01.2014**

(51) Int Cl.:
*A61L 15/24* (2006.01)     *A61L 15/22* (2006.01)
*A61L 15/60* (2006.01)     *C08F 6/00* (2006.01)
*C08L 33/02* (2006.01)     *C08F 220/06* (2006.01)

(86) Internationale Anmeldenummer:
**PCT/EP2014/050988**

(87) Internationale Veröffentlichungsnummer:
**WO 2014/118025 (07.08.2014 Gazette 2014/32)**

(54) **VERFAHREN ZUR ENTFERNUNG VON RESTMONOMEREN AUS WASSERABSORBIERENDEN POLYMERPARTIKELN**

METHOD FOR REMOVAL OF RESIDUAL MONOMERS FROM WATER-ABSORBING POLYMER PARTICLES

PROCÉDÉ D'ÉLIMINATION DE MONOMÈRES RÉSIDUELS PRÉSENTS DANS DES PARTICULES POLYMÈRES HYDROABSORBANTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.01.2013  EP 13153279**

(43) Veröffentlichungstag der Anmeldung:
**09.12.2015  Patentblatt 2015/50**

(73) Patentinhaber: **BASF SE**
**67056 Ludwigshafen am Rhein (DE)**

(72) Erfinder:
• **BAUER, Stephan**
**65239 Hochheim (DE)**
• **HERFERT, Norbert**
**63674 Altenstadt (DE)**
• **KÖNIG, Lydia**
**67376 Harthausen (DE)**
• **HAGEN, Yvonne**
**67165 Waldsee (DE)**
• **ROMANI FERNANDEZ, Xiana**
**76137 Karlsruhe (DE)**
• **SCHÖPPLER, Marcus**
**68804 Altlußheim (DE)**

(74) Vertreter: **BASF IP Association**
**BASF SE**
**G-FLP-C006**
**67056 Ludwigshafen (DE)**

(56) Entgegenhaltungen:
**WO-A1-2008/092843     WO-A1-2010/018143**
**US-A1- 2011 059 329**

**Beschreibung**

Die vorliegende Erfindung betrifft ein Verfahren zur Entfernung von Restmonomeren aus wasserabsorbierenden Polymerpartikeln, wobei die wasserabsorbierenden Polymerpartikel in einem Mischer mit rotierenden Mischwerkzeugen bei einer Temperatur von mindestens 60°C in Gegenwart von Wasser und eines Tensids thermisch nachbehandelt werden.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Wasserabsorbierende Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet. Wasserabsorbierende Polymere werden auch als "superabsorbent polymers" bzw. "Superabsorber" bezeichnet.

WO 2008/095901 A1 beschreibt ein Verfahren zur Entfernung von Restmonomeren durch thermische Nachbehandlung mit Wasserdampf im fluidisierten Zustand.

WO 2011/117215 A1 beschreibt ein Verfahren zur Entfernung von Restmonomeren aus wasserabsorbierenden Polymerpartikeln, wobei die wasserabsorbierenden Polymerpartikel in einem Mischer mit rotierenden Mischwerkzeugen bei einer Temperatur von mindestens 60°C in Gegenwart von Wasserdampf thermisch nachbehandelt werden.

Aufgabe der vorliegenden Erfindung war die Bereitstellung eines verbesserten und schonenden Verfahrens zur Entfernung von Restmonomeren aus wasserabsorbierenden Polymerpartikeln. Insbesondere soll bei höheren Wassergehalten die Entstehung von Agglomeraten verhindert oder zumindest zurückgedrängt werden.

Als Agglomerate werden Partikel bezeichnet, die aus mindestens zwei aneinander verbundenen Primärpartikeln bestehen und während des thermischen Nachbehandlungsschritts gebildet werden und eine Partikelgröße von größer 850 $\mu$m aufweisen.

Gelöst wurde die Aufgabe durch ein Verfahren zur Entfernung von Restmonomeren aus wasserabsorbierenden Polymerpartikeln, wobei die wasserabsorbierenden Polymerpartikel in einem Mischer mit rotierenden Mischwerkzeugen thermisch nachbehandelt werden, die wasserabsorbierenden Polymerpartikel während der thermischen Nachbehandlung eine Temperatur von mindestens 60°C und einen Wassergehalt von mindestens 8 Gew.-% aufweisen, wobei vor oder während der thermischen Nachbehandlung Wasser oder eine wässrige Lösung und/oder während der thermischen Nachbehandlung ein Wasserdampf enthaltender Gasstrom zugesetzt wird, dadurch gekennzeichnet, dass die thermische Nachbehandlung in Gegenwart mindestens eines Tensids durchgeführt wird, das mindestens eine Tensid eine polare und eine unpolare Gruppe aufweist, die polare und die unpolare Gruppe des Tensids nicht über eine Carbonsäureester-Gruppe verbunden sind, die polare Gruppe mindestens eine Hydroxyl-Gruppe, eine kationische Gruppe oder eine anionische Gruppe aufweist und die unpolare Gruppe eine $C_4$- bis $C_{20}$-Alkylkette aufweist.

Die Temperatur der wasserabsorbierenden Polymerpartikel beträgt während der thermischen Nachbehandlung vorzugsweise von 60 bis 140°C, besonders bevorzugt von 70 bis 125°C, ganz besonders von 80 bis 110°C.

Der Wassergehalt der wasserabsorbierenden Polymerpartikel beträgt während der thermischen Nachbehandlung vorzugsweise von 8 bis 50 Gew.-%, besonders bevorzugt von 12 bis 30 Gew.-%, ganz besonders bevorzugt von 15 bis 25 Gew.-%.

Als polare Gruppen sind kationische Gruppen bevorzugt. Weiterhin sind Tensidmischungen bevorzugt. In den Tensidmischungen sind bevorzugt Tenside mit unterschiedlichen linearen und/oder verzweigten Alkylketten als unpolare Gruppen enthalten. Besonders bevorzugt sind Tensidmischungen, deren Alkylketten sich vom Kokosnussöl ableiten, d.h. Tensidmischungen mit $C_6$-, $C_8$-, $C_{10}$-, $C_{12}$-, $C_{14}$-, $C_{16}$- und $C_{18}$-Alkylketten.

Der vorliegenden Erfindung liegt die Erkenntnis zu Grunde, dass sich die Entstehung von Agglomeraten durch Zusatz bestimmter Tenside als Deggomerationshilfsmittel senken lässt. Wesentlich scheint dabei zu sein, dass die polare und die unpolare Gruppe des Tensids auch unter den Bedingungen der thermischen Nachbehandlung verbunden bleiben. Tenside, die an dieser Stelle eine hydrolyseempfindliche Carbonsäureester-Gruppe aufweisen sind ungeeignet. Geeignete Tenside oder Tensidmischungen sind:

a) Alkyl- und Polyglykoside, beispielsweise Laurylglycosid, Cocoglycosid und Tensidmischungen, die diese Verbindungen enthalten. Derartige Tenside sind unter den Handelsnamen Plantacare® (BASF SE) und Euperlan® (BASF SE) und Lutensol®GD (BASF SE) erhältlich. Beispielhaft zu nennen sind Cocoglycosid (Plantacare®818UP, BASF SE, CAS-Nr.110615-47-9/68515-73-1), Laurylglycosid (Plantacare®1200UP, BASF SE, CAS-Nr. CAS-Nr.110615-47-9), Mischungen aus Laurylglycosid und Cocomidpropylbetain (Plantacare®K55, BASF SE, CAS-Nr. CAS-Nr.110615-47-9/61789-40-0), Mischungen aus Natriumlaurethsulfat and Laurylglycosid (Plantacare®PS10, BASF SE, CAS-Nr. CAS-Nr.110615-47-9/68891-38-3), perlglanzartige Wachsdispersionen mit Laurylglycosid und Stearylcitrat (Euperlan®Green, BASF SE, CAS-Nr. CAS-Nr.110615-47-9/1337-33-4), Stryrol/Acrylat-Dispersion mit Cocoglycosid (Euperlan®PCO, BASF SE).
b) Ethoxylierte Alkylalkohole der allgemeinen Struktur $H_{2n+1}C_nO(C_2H_4O)_nH$, insbesondere Fett-, Oxo-, Guerbeta-

kolhole. Derartige Tenside sind beispielsweise unter den Handelsnamen Lutensol®AT, AO, TO, XP, XL, XA, ON (BASF SE) erhältlich.

c) Ethoxylierte Alkylphenole, beispielsweise Lutensol®AP (BASF SE).

d) Ethoxyliertes Rizinusöl, bespielsweise Emulan® (BASF SE).

e) Ethyoxilierte Alkylamide, beispielsweise Oleyl-, Coco-, Olsäureamid. Derartige Tenside sind unter den Handelsnamen Lutensol®FA und FSA (BASF SE) erhältlich.

f) Kationische Tenside, beispielweise Benzalchoniumchlorid, Benzyldimethyldodecylammoniumchlorid, Benzyldimethylammoniumchlorid, Miristalkoniumchlorid, Cetalkoniumchlorid, Benzyldimethylstearylammoniumchlorid, Benzyldimethylstearylammoniumchlorid, Didecyldimethylammoniumchlorid, Didodecyldimethylammoniumchlorid, Dimethylditetradecylammoniumchlorid, Dihexadecyldimethylammoniumchlorid, Dimethyldioctadecylammoniumchlorid, Ditallowdimethylammoniumchlorid, Hexadecyl-(2-hydroxyethyl)dimethylammonium dihydrogenphosphat, ganz bevorzugt ist Hexadecyl-(2-hydroxyethyl)dimethylammonium dihydrogenphosphat, erhältlich unter dem Handelsnamen Luviquat®Mono CP (BASF SE).

Ganz bevorzugt sind Laurylglycosid, Cocoglycosid, Ditallowdimethylammoniumchlorid und Hexadecyl-(2-hydroxyethyl)dimethylammonium dihydrogenphosphat.

Das Tensid wird in einer Menge von vorzugsweise 0,0001 bis 0,1 Gew.-%, besonders bevorzugt 0,0002 bis 0,05 Gew.-%, ganz besonders bevorzugt 0,0005 bis 0,01 Gew.-%, jeweils bezogen auf die wasserabsorbierenden Polymerpartikel, eingesetzt.

Das Tensid senkt die Oberflächenspannung des wässrigen Extrakts und kann so die Leckrate von Windeln erhöhen. Das Tensid wird daher vorteilhaft so dosiert, dass die Oberflächenspannung des wässrigen Extrakts um höchstens 10 mN/m sinkt. Zur Messung der Oberflächenspannung des wässrigen Extrakts werden 2g wasserabsorbierende Polymerpartikel in einem Becherglas mit so viel Waser gequollen, dass nach der vollständigen Quellung eine etwa 1 cm dicke Wassersicht über dem entstandenen Hydrogel steht. Anschließend wird die Oberflächenspannung des Wassers mit einem Tensiometer gemessen.

Vor oder während der thermischen Nachbehandlung kann Wasser oder eine wässrige Lösung zugesetzt werden, vorzugsweise von 0,01 bis 25 Gew.-%, besonders bevorzugt von 0,05 bis 20 Gew.-%, ganz besonders bevorzugt von 0,1 bis 15 Gew.-%, jeweils bezogen auf die wasserabsorbierenden Polymerpartikel. Die Temperatur des Wasser oder der wässrigen Lösung beträgt vorzugsweise von 1 bis 99°C, besonders bevorzugt von 20 bis 80°C, ganz besonders bevorzugt von 40 bis 70°C. Die Temperatur der wasserabsorbierenden Polymerpartikel vor dem Aufsprühen des Wassers oder der wässrigen Lösung liegt dabei im Bereich von vorzugsweise 1 bis 160°C, bevorzugt 20 bis 120°C, besonders bevorzugt 30 bis 100°C, ganz besonders bevorzugt 60 bis 90°C. Vorteilhaft wird das Wasser oder die wässrige Lösung mittels mehrerer Düsen in einem geeigneten Mischer aufgebracht.

Während der thermischen Nachbehandlung kann ein Wasserdampf enthaltender Gasstrom zugesetzt werden. Der Gasstrom enthält vorzugsweise von 0,01 bis 2,0 kg Wasserdampf pro kg trockenes Gas, besonders bevorzugt von 0,05 bis 1,0 kg Wasserdampf pro kg trockenes Gas, ganz besonders bevorzugt von 0,1 bis 0,5 kg Wasserdampf pro kg trockenes Gas.

Die mittlere Verweilzeit im Mischer während der thermischen Nachbehandlung beträgt vorzugsweise von 10 bis 120 Minuten, bevorzugt von 12 bis 100 Minuten, besonders bevorzugt von 15 bis 90 Minuten, ganz besonders bevorzugt von 20 bis 40 Minuten.

Das zur thermischen Nachbehandlung eingesetzte Gasvolumen beträgt in einem diskontinuierlichen Mischer vorzugsweise von 0,01 bis 5 $Nm^3$/h, besonders bevorzugt von 0,05 bis 2 $Nm^3$/h, ganz besonders bevorzugt von 0,1 bis 0,5 $Nm^3$/h, jeweils pro kg wasserabsorbierende Polymerpartikel und in einem kontinuierlichen Mischer vorzugsweise von 0,01 bis 5 $Nm^3$/h, besonders bevorzugt von 0,05 bis 2 $Nm^3$/h, ganz besonders bevorzugt von 0,1 bis 0,5 $Nm^3$/h, jeweils pro kg/h durchgesetzter wasserabsorbierender Polymerpartikel. Das Gasvolumen ist hierbei das auf Standardbedingungen (0°C; 1.013,25 hPa) korrigierte Gasvolumen.

Die übrigen Bestandteile des Gases sind vorzugsweise Stickstoff, Kohlendioxid, Argon, Xenon, Krypton, Neon, Helium, Luft oder Luft/Stickstoff-Gemische, besonders bevorzugt Stickstoff oder Luft/Stickstoff-Gemische mit weniger als 10 Vol.-% Sauerstoff.

Die Anwesenheit von Sauerstoff kann zu Verfärbungen der wasserabsorbierenden Polymerpartikel führen. Luft ist dagegen besonders kostengünstig.

Im erfindungsgemäßen Verfahren können alle dem Fachmann bekannten diskontinuierlichen und kontinuierlichen Mischer mit rotierenden Mischwerkzeugen, wie Schneckenmischer, Scheibenmischer, Schraubenbandmischer und Schaufelmischer eingesetzt werden. Geeignete Mischer sind beispielsweise Becker Shovel Mixer (Gebr. Lödige Maschinenbau GmbH; Paderborn; DE), Nara Paddle Mixer (NARA Machinery Europe; Frechen; DE), Pflugschar® Mischer (Gebr. Lödige Maschinenbau GmbH; Paderborn; DE), Vrieco-Nauta Continuous Mixer (Hosokawa Micron BV; Doetinchem; NL), Processall Mixmill Mixer (Processall Incorporated; Cincinnati; US) und Ruberg Durchlaufmischer (Ruberg GmbH & Co KG; Nieheim; DE). Im erfindungsgemäßen Verfahren werden vorzugsweise Becker Shovel Mixer, Pflugschar® Mischer und Ruberg Durchlaufmischer zur thermischen Nachbehandlung verwendet.

Mischer mit rotierenden Mischwerkzeugen werden gemäß der Lage der Rotationsachse in Vertikalmischer und Horizontalmischer unterteilt. Vorteilhaft werden für das erfindungsgemäße Verfahren Horizontalmischer verwendet.

Horizontalmischer im Sinne dieser Erfindung sind Mischer mit rotierenden Mischwerkzeugen deren Lage der Rotationsachse zur Produktstromrichtung um weniger als 20°, vorzugsweise um weniger als 15°, besonders bevorzugt um weniger als 10°, ganz besonders bevorzugt um weniger als 5°, von der Horizontalen abweicht.

Für Mischer mit horizontal gelagertem Mischwerkzeugen ist die Froude-Zahl wie folgt definiert:

$$Fr = \frac{\omega^2 r}{g}$$

mit

r:     Radius des Mischwerkzeugs
ω:     Kreisfrequenz
g:     Erdbeschleunigung

Die Froude-Zahl beträgt vorzugsweise von 0,1 bis 6, besonders bevorzugt von 0,15 bis 3, ganz besonders bevorzugt von 0,2 bis 1.

Die Innenwand des Mischers weist gegenüber Wasser einen Randwinkel von vorzugsweise weniger als 70°, besonders bevorzugt von weniger als 60°, ganz besonders bevorzugt von weniger als 50°, auf. Der Randwinkel ist ein Maß für das Benetzungsverhalten und wird gemäß DIN 53900 gemessen.

Vorteilhaft werden im erfindungsgemäßen Verfahren Mischer eingesetzt, deren produktberührte Innenwand aus einem nichtrostenden Stahl ist. Nichtrostende Stähle weisen üblicherweise einen Chromgehalt von 10,5 bis 13 Gew.-% Chrom auf. Der hohe Chromanteil führt zu einer schützenden Passivierung aus Chromdioxid an der Stahloberfläche. Weitere Legierungsbestandteile erhöhen die Korrosionsbeständigkeit und verbessern die mechanischen Eigenschaften.

Besonders geeignete Stähle sind austenitische Stähle mit beispielsweise mindestens 0,08 Gew.-% Kohlenstoff. Vorteilhaft enthalten die austenitischen Stähle neben Eisen, Kohlenstoff, Chrom, Nickel und optional Molybdän noch weitere Legierungsbestandteile, vorzugsweise Niob oder Titan.

Die bevorzugten nichtrostenden Stähle sind Stähle mit der Werkstoffnummer 1.43xx oder 1.45xx gemäß der DIN EN 10020, wobei xx eine natürliche Zahl zwischen 0 und 99 sein kann. Besonders bevorzugte Werkstoffe sind die Stähle mit den Werkstoffnummern 1.4301, 1.4541 und 1.4571, insbesondere Stahl mit der Werkstoffnummer 1.4301.

Vorteilhaft ist die produktberührte Innenwand des Mischers poliert oder elektropoliert. Polierte nichtrostende Stahloberflächen weisen eine niedrigere Rauhigkeit und einen niedrigeren Randwinkel gegenüber Wasser auf als matte oder aufgeraute Stahloberflächen.

In einer Ausführungsform des Verfahrens ist der Mischer mit einer Antihaftbeschichtung ausgerüstet, wie zum Beispiel mit Polytetrafluorethylen (PTFE), einer Nickel-Phosphor-Legierung mit eingelagerten Polytetrafluorethylenpartikeln (Ni-PTFE), Polyvinylidenfluorid (PVDF) oder einem anderen geeigneten Polymer, beispielsweise mit hydrophoben Polymeren, wie Silikonen, oder ein Chemical Vapor Deposition (CVD) Coating. Letzteres wird in dem Conference Paper von S.Montgomery, D.Kennedy, N.O'Dowd, "PVD and CVD Coating for the Metal Forming Industry", Matrib 2010, Kroatien, beschrieben.

Im Folgenden wird die Herstellung der wasserabsorbierenden Polymerpartikel beschrieben:
Die wasserabsorbierenden Polymerpartikel werden beispielsweise durch Polymerisation einer Monomerlösung, enthaltend

    a) mindestens ein ethylenisch ungesättigtes, säuregruppentragendes Monomer, das zumindest teilweise neutralisiert sein kann,
    b) mindestens einen Vernetzer,
    c) mindestens einen Initiator,
    d) optional ein oder mehrere mit den unter a) genannten Monomeren copolymerisierbare ethylenisch ungesättigte Monomere,
    e) optional ein oder mehrere wasserlösliche Polymere oder Copolymere,

hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure, Vinylsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Säuregruppen der Monomere a) sind üblicherweise teilweise neutralisiert, vorzugsweise zu mindestens 25 mol-%, bevorzugt zu 50 bis 80 mol-%, besonders bevorzugt 60 bis 75 mol-%, ganz besonders bevorzugt 65 bis 72 mol%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen. Üblicherweise wird die Neutralisation durch Einmischung des Neutralisationsmittels als wässrige Lösung, als Schmelze, oder bevorzugt auch als Feststoff erreicht. Beispielsweise kann Natriumhydroxid mit einem Wasseranteil deutlich unter 50 Gew.-% als wachsartige Masse mit einem Schmelzpunkt oberhalb 23°C vorliegen. In diesem Fall ist eine Dosierung als Stückgut oder Schmelze bei erhöhter Temperatur möglich.

Optional können der Monomerlösung oder ihren Ausgangsstoffen ein oder mehrere Chelatbildner zur Maskierung von Metallionen, wie beispielsweise Eisen, zwecks Stabilisierung zugesetzt werden. Geeignete Chelatbildner sind beispielsweise Alkalicitrate, Zitronensäure, Alkalitartrate, Pentanatriumtriphosphat, Ethylendiamintetraazetat, Nitrilotriessigsäure, sowie alle unter dem Namen Trilon® bekannten Chelatbildner, wie beispielsweise Trilon® C (Pentanatriumdiethylentriaminpentaazetat), Trilon® D (Trinatrium-(hydroxyethyl)-ethylen-diamintriazetat), sowie Trilon® M (Methylglycindiessigsäure).

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraallyloxyethan, Methylenbismethacrylamid, 15-fach ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 20-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 g/cm$^2$ (AUL0.3psi) durchläuft ein Maximum.

Als Initiatoren c) können sämtliche unter den Polymerisationsbedingungen in Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; DE) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Maleinsäure, Fumarsäure, Dimethylaminoethylmethacrylat, Dimethylaminoethylacrylat, Dimethylaminopropylacrylat, Diethylaminopropylacrylat, Dimethylaminoethylmethacrylat und Diethylaminoethylmethacrylat.

Als wasserlösliche Polymere oder Copolymere e) können Polyvinylalkohol, Polyvinylpyrrolidon, Polyvinylamine, Polyallylamine, Polyethylenimine, Polyacrylamid, lineare hydrophobe Polyurethane, Stärke, Stärkederivate, modifizierte Cellulose, wie Methylcellulose oder Hydroxyethylcellulose, Gelatine, Polyethylenglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren (Gelpolymerisation). Im Kneter wird das bei der Polymerisation einer wässrigen Monomerlösung entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Bei Verwendung von Knetreaktoren oder Bandreaktoren ist es möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das erhaltene Polymergel wird vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA (European Disposables and Nonwovens Association) empfohlenen Testmethode Nr. WSP 230.2-05 "Moisture Content" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 μm, besonders bevorzugt von 250 bis 600 μm, ganz besonders von 300 bis 500 μm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 μm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Die geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. können anderweitig beschichtet sein, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 μm, beträgt vorzugsweise mindestens 90 Gew.-%, besonders bevorzugt mindestens 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Es ist aber auch möglich eine wässrige Monomerlösung zu vertropfen und die erzeugten Tropfen in einem erwärmten Trägergasstrom zu polymerisieren. Hierbei können die Verfahrensschritte Polymerisation und Trocknung zusammengefasst werden, wie in WO 2008/052971 A1 und WO 2011/026876 A1 beschrieben.

Die Monomerlösung wird hierzu mittels mindestens einer Bohrung unter Ausbildung von Tropfen in den Reaktionsraum dosiert. Die Bohrungen können sich beispielsweise in einer Vertropferplatte befinden.

Eine Vertropferplatte ist eine Platte mit mindestens einer Bohrung, wobei die Flüssigkeit von oben durch die Bohrung tritt. Die Vertropferplatte bzw. die Flüssigkeit kann in Schwingungen versetzt werden, wodurch an der Unterseite der Vertropferplatte je Bohrung eine idealerweise monodisperse Tropfenkette erzeugt wird. In einer bevorzugten Ausführungsform wird die Vertropferplatte nicht angeregt.

Die Anzahl und die Größe der Bohrungen werden gemäß der gewünschten Kapazität und Tropfengröße ausgewählt. Der Tropfendurchmesser beträgt dabei üblicherweise das 1,9-fache des Durchmessers der Bohrung. Wichtig ist hierbei, dass die zu vertropfende Flüssigkeit nicht zu schnell durch die Bohrung tritt bzw. der Druckverlust über die Bohrung nicht zu groß ist. Ansonsten wird die Flüssigkeit nicht vertropft, sondern der Flüssigkeitsstrahl wird infolge der hohen kinetischen Energie zerrissen (versprüht). Die Reynoldszahl bezogen auf den Durchsatz pro Bohrung und den Bohrungsdurchmesser ist vorzugsweise kleiner als 2.000, bevorzugt kleiner 1.600, besonders bevorzugt kleiner 1.400, ganz besonders bevorzugt kleiner 1.200.

Die Unterseite der Vertropferplatte weist gegenüber Wasser einen Kontaktwinkel (Randwinkel) von vorzugsweise mindestens 60°, besonders bevorzugt mindestens 75°, ganz besonders bevorzugt mindestens 90°, auf.

Der Kontaktwinkel ist ein Maß für das Benetzungsverhalten von Wasser, gegenüber einer Oberfläche und kann mit üblichen Methoden bestimmt werden, beispielsweise gemäß ASTM D 5725. Ein niedriger Kontaktwinkel bedeutet eine gute und ein hoher Kontaktwinkel eine schlechte Benetzung.

Es ist aber auch möglich, dass die Vertropferplatte aus einem Material mit einem niedrigeren Kontaktwinkel gegenüber Wasser besteht, beispielsweise einem Stahl mit der Werkstoffnummer 1.4571, und mit einem Material mit einem größeren Kontaktwinkel gegenüber Wasser beschichtet wird.

Geeignete Beschichtungen sind beispielsweise fluorhaltige Polymere, wie Perfluoralkoxyethylen, Polytetrafluorethylen, Ethylen-Chlortrifluorethylen-Copolymere, Ethylen-Tetrafluorethylen-Copolymere und fluoriertes Polyethylen.

Die Beschichtungen können auch als Dispersion aufgebracht werden, wobei das Dispergiermittel bei der anschließenden Erwärmung verdampft. Ein derartiges Verfahren wird beispielsweise in US 3,243,321 beschrieben.

Weitere Beschichtungsverfahren sind unter dem Stichwort "Thin Films" in der elektronischen Version von "Ullmann's Encyclopedia of Industrial Chemistry" zu finden.

Die Beschichtung kann aber auch eine durch chemische Vernickelung hergestellte Nickelschicht sein.

Infolge der schlechten Benetzbarkeit der Vertropferplatte werden monodisperse Tropfen mit enger Tropfengrößenverteilung erhalten.

Die Vertropferplatte weist vorzugsweise mindestens 5, besonders bevorzugt mindestens 25, ganz besonders bevorzugt mindestens 50, und vorzugsweise bis zu 750, besonders bevorzugt bis zu 500, ganz besonders bevorzugt bis zu 250, Bohrungen auf. Der Durchmesser der Bohrungen wird entsprechend der gewünschten Tropfengröße ausgewählt.

Der Durchmesser der Bohrungen beträgt vorzugsweise von 50 bis 500 $\mu$m, besonders bevorzugt von 100 bis 300 $\mu$m, ganz besonders bevorzugt von 150 bis 250 $\mu$m.

Die Temperatur der Monomerlösung beim Durchtritt durch die Bohrungen beträgt vorzugsweise von 5 bis 80°C, besonders bevorzugt von 10 bis 70°C, ganz besonders bevorzugt von 30 bis 60°C.

Der Abstand der Bohrungen beträgt vorzugsweise 10 bis 50 mm, besonders bevorzugt 12 bis 40 mm, ganz besonders bevorzugt 15 bis 30 mm. Zu geringe Abstände führen zur Bildung von Agglomeraten.

Der Polymerisationsreaktor wird von einem Trägergas durchströmt. Dabei kann das Trägergas im Gleichstrom oder im Gegenstrom zu den frei fallenden Tropfen der Monomerlösung durch den Reaktionsraum geführt werden, bevorzugt im Gleichstrom, d.h. von unten nach oben. Vorzugsweise wird das Trägergas nach einem Durchgang zumindest teilweise, bevorzugt zu mindestens 50%, besonders bevorzugt zu mindestens 75%, als Kreisgas in den Reaktionsraum zurückgeführt. Üblicherweise wird eine Teilmenge des Trägergases nach jedem Durchgang ausgeschleust, vorzugsweise bis zu 10%, besonders bevorzugt bis zu 3%, ganz besonders bevorzugt bis zu 1%.

Der Sauerstoffgehalt des Trägergases beträgt vorzugsweise von 0,5 bis 15 Vol.-%, besonders bevorzugt von 1 bis 10 Vol.-%, ganz besonders bevorzugt von 2 bis 7 Gew.-%.

Das Trägergas enthält neben Sauerstoff vorzugsweise Stickstoff. Der Stickstoffgehalt des Trägergases beträgt vorzugsweise mindestens 80 Vol.-%, besonders bevorzugt mindesten 90 Vol.-%, ganz besonders bevorzugt mindestens 95 Vol.-%. Weitere geeignete Trägergase sind Kohlendioxid, Argon, Xenon, Krypton, Neon und Helium. Es können auch Gasmischungen verwendet werden. Das Trägergas kann auch mit Wasserdampf und/oder Acrylsäuredämpfen beladen werden.

Die Gasgeschwindigkeit wird vorzugsweise so eingestellt, dass die Strömung im Polymerisationsreaktor gerichtet ist, beispielsweise liegen keine der allgemeinen Strömungsrichtung entgegen gesetzte Konvektionswirbel vor, und beträgt üblicherweise 0,1 bis 2,5 m/s, vorzugsweise 0,3 bis 1,5 m/s bevorzugt von 0,5 bis 1,2 m/s, besonders bevorzugt 0,6 bis 1,0 m/s, ganz besonders bevorzugt 0,7 bis 0,9 m/s.

Das den Reaktor durchströmende Trägergas wird zweckmäßigerweise vor dem Reaktor auf die Reaktionstemperatur vorgewärmt.

Vorteilhaft wird die Gaseintrittstemperatur so geregelt, dass die Gasaustrittstemperatur, d.h. die Temperatur mit der das Trägergas den Reaktionsraum verlässt üblicherweise von 90 bis 150°C, vorzugsweise von 100 bis 140°C, bevorzugt von 105 bis 135°C, besonders bevorzugt von 110 bis 130°C, ganz besonders bevorzugt von 115 bis 125°C, beträgt.

Die Reaktion kann im Überdruck oder im Unterdruck durchgeführt werden, ein Unterdruck von bis zu 100 mbar gegenüber dem Umgebungsdruck ist bevorzugt.

Das Reaktionsabgas, d.h. das den Reaktionsraum verlassende Gas, kann beispielsweise in einem Wärmeaustauscher abgekühlt werden. Dabei kondensieren Wasser und nicht umgesetztes Monomer a). Danach kann das Reaktionsabgas zumindest teilweise wieder aufgewärmt und als Kreisgas in den Reaktor zurückgeführt werden. Ein Teil des Reaktionsabgases kann ausgeschleust und durch frisches Trägergas ersetzt werden, wobei im Reaktionsabgas enthaltenes Wasser und nicht umgesetzte Monomere a) abgetrennt und rückgeführt werden können.

Besonders bevorzugt ist ein Wärmeverbund, d.h., ein Teil der Abwärme beim Abkühlen des Abgases wird zum Aufwärmen des Kreisgases verwendet.

Die Reaktoren können begleitbeheizt werden. Die Begleitheizung wird dabei so eingestellt, dass die Wandtemperatur mindestens 5°C oberhalb der Reaktorinnentemperatur liegt und die Kondensation an den Reaktorwänden zuverlässig vermieden wird.

Das Reaktionsprodukt wird anschließend thermisch nachbehandelt und optional bis auf den gewünschten Wassergehalt getrocknet.

Die wasserabsorbierenden Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder β-Hydroxyalkylamide, wie in DE 102 04 938 A1 und US 6,239,230 beschrieben.

Des Weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind Ethylenkarbonat, 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 5 Gew.-%, besonders bevorzugt 0,02 bis 2,5 Gew.-%, besonders bevorzugt 0,03 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern Kationen, vorzugsweise polyvalente Kationen, auf die Partikeloberfläche aufgebracht.

Die im erfindungsgemäßen Verfahren einsetzbaren Kationen bzw. polyvalenten Kationen sind beispielsweise Ammonium, zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat, Citrat, Lactat, Lactamid, Glycinat, Glykolat, 3-Hydroxypropionat, Methansulfonat, Methansulfonamid, sowie in der Patentanmeldung WO 2012/045705 A1 beschriebene Gegenionen, und deren Gemische, möglich.

Aluminiumsulfat, Aluminiumlaktat und Zirkoniumacetat sind bevorzugt. Außer Metallsalzen können auch Polyamine als polyvalente Kationen eingesetzt werden.

In einer weiteren Ausführungsform können organische Salze aus den Kationen und den Gegenionen durch getrennte Zugabe in den verschieden Verfahrensschritten, wie vor, während oder nach der Oberflächennachvernetzung erzeugt werden. Die Zugabe kann in beliebiger Reihenfolge erfolgen. Besonders bevorzugte sind Kationen sind Ammonium, sowie die Kationen des Aluminiums und des Zirkoniums. Als organische Gegenionen kommen vorzugsweise Carboxlyate, wie Acetat, Lactat, Glykolat, Citrat, in Form der freien Säure und der Salze, sowie Carboxamide, wie Lactamid, Glycinat, in Betracht.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; DE), Vrieco-Nauta Continuous Mixer (Hosokawa Micron BV; Doetinchem; NL), Processall Mixmill Mixer (Processall Incorporated; Cincinnati; US) und Schugi Flexomix® (Hosokawa Micron BV; Doetinchem; NL). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser, Propylenglykol/Wasser und Ethylenkarbonat/Wasser, wobei das Mischungsmassenverhältnis vorzugsweise von 20:80 bis 40:60 beträgt.

Als Tenside werden hierbei vorzugsweise die erfindungsgemäßen Tenside und Tensidmischungen verwendet.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; DE), Hosokawa Bepex® Disc Dryer (Hosokawa Micron GmbH; Leingarten; DE) und Nara Paddle Dryer (NARA Machinery Europe; Frechen; DE). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, Tenside, wie Span® 20, und die erfindungsgemäßen Tenside und Tensidmischungen.

Das erfindungsgemäße Verfahren ermöglicht die Herstellung wasserabsorbierender Polymerpartikel mit sehr niedrigem Gehalt an Restmonomeren.

Die gemäß dem erfindungsgemäßen Verfahren erhältlichen wasserabsorbierenden Polymerpartikel weisen eine Zentrifugenretentionskapazität (CRC) von typischerweise mindestens 15 g/g, vorzugsweise mindestens 20 g/g, bevorzugt mindestens 25 g/g, besonders bevorzugt mindestens 30 g/g, ganz besonders bevorzugt mindestens 35 g/g, auf. Die Zentrifugenretentionskapazität (CRC) der wasserabsorbierenden Polymerpartikel beträgt üblicherweise weniger als 100 g/g.

Die gemäß dem erfindungsgemäßen Verfahren erhältlichen wasserabsorbierenden Polymerpartikel weisen einen Gehalt an Restmonomeren von typischerweise weniger als 0,5 Gew.-%, vorzugsweise weniger als 0,3 Gew.-%, besonders bevorzugt weniger als 0,2 Gew.-%, ganz besonders bevorzugt von weniger als 0,15 Gew.-%, auf.

Die gemäß dem erfindungsgemäßen Verfahren erhältlichen wasserabsorbierenden Polymerpartikel weisen einen Feuchtegehalt von vorzugsweise mindestens 5 Gew.-%, besonders bevorzugt von mindestens 10 Gew.-%, ganz besonders bevorzugt von mindestens 14 Gew.-%, auf.

Der mittlere Durchmesser der gemäß dem erfindungsgemäßen Verfahren erhältlichen wasserabsorbierenden Polymerpartikel beträgt vorzugsweise mindestens 200 µm, besonders bevorzugt von 250 bis 600 µm, ganz besonders von 300 bis 500 µm, wobei der Partikeldurchmesser durch Lichtstreuung bestimmt werden kann und den volumengemittelten mittleren Durchmesser bedeutet. 90% der Polymerpartikel weisen einen Durchmesser von vorzugsweise 100 bis 800 µm, besonders bevorzugt von 150 bis 700 µm, ganz besonders bevorzugt von 200 bis 600 µm, auf.

Die Herstellung der Hygieneartikel wird in der Monographie "Modern Superabsorbent Polymer Technology", F.L. Buchholz und AT. Graham, Wiley-VCH, 1998, Seiten 252 bis 258, beschrieben.

Die Hygieneartikel enthalten üblicherweise eine wasserundurchlässige Rückseite, eine wasserdurchlässige Oberseite und dazwischen einen absorbierenden Kern aus den erfindungsgemäßen wasserabsorbierenden Polymerpartikeln und Fasern, vorzugsweise Cellulose. Der Anteil der erfindungsgemäßen wasserabsorbierenden Polymerpartikel im absorbierenden Kern beträgt vorzugsweise 20 bis 100 Gew.-%, bevorzugt 50 bis 100 Gew.-%.

Die wasserabsorbierenden Polymerpartikel werden mittels der nachfolgend beschriebenen Testmethoden geprüft.

Methoden:

Die Messungen sollten, wenn nicht anders angegeben, bei einer Umgebungstemperatur von 23 $\pm$ 2 °C und einer relativen Luftfeuchte von 50 $\pm$ 10 % durchgeführt werden. Die wasserabsorbierenden Polymere werden vor der Messung gut durchmischt.

Restmonomer

Der Gehalt an Restmonomer der wasserabsorbierenden Polymerpartikel wird gemäß der von der EDANA empfohlenen Testmethode WSP Nr. 210.2-05 "Residual Monomers" bestimmt.

Feuchtegehalt

Der Feuchtegehalt der wasserabsorbierenden Polymerpartikel wird gemäß der von der EDANA empfohlenen Testmethode Nr. WSP 230.2-05 "Mass Loss Upon Heating" bestimmt.

Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

Absorption unter einem Druck von 21,0 g/cm$^2$

Die Absorption unter einem Druck von 21,0 g/cm$^2$ (AUL0.3psi) der wasserabsorbierenden Polymerpartikel wird gemäß der von der EDANA empfohlenen Testmethode Nr. WSP 242.2-05 "Absorption Under Pressure, Gravimetric Determination" bestimmt.

Absorption unter einem Druck von 0,0 g/cm$^2$

Die Absorption unter einem Druck von 0,0 g/cm$^2$ (AUL0.0psi) wird analog der von der EDANA empfohlenen Testmethode Nr. WSP 242.2-05 "Absorption Under Pressure, Gravimetric Determination" bestimmt, wobei statt eines Drucks von 21,0 g/cm$^2$ (AUL0.3psi) ein Druck von 0,0 g/cm$^2$ (AUL0.0psi) eingestellt wird.

Absorption unter einem Druck von 49,2 g/cm$^2$

Die Absorption unter einem Druck von 49,2 g/cm$^2$ (AUL0.7psi) wird analog der von der EDANA empfohlenen Testmethode Nr. WSP 242.2-05 "Absorption Under Pressure, Gravimetric Determination" bestimmt, wobei statt eines Drucks von 21,0 g/cm$^2$ (AUL0.3psi) ein Druck von 49,2 g/cm$^2$ (AUL0.7psi) eingestellt wird.

Schüttgewicht

Das Schüttgewicht wird gemäß der von der EDANA empfohlenen Testmethode Nr. WSP 260.2-05 "Density, Gravimetric Determination" bestimmt.

Extrahierbare

Der Gehalt an extrahierbaren Bestandteilen der wasserabsorbierenden Polymerpartikel wird analog der von der EDANA empfohlenen Testmethode Nr. WSP 270.2-05 "Extractable" bestimmt. Die Extraktionszeit beträgt 16 Stunden.

Anquellgeschwindigkeit

Zur Bestimmung der Anquellgeschwindigkeit (FSR) werden 1,00 g (= W1) der wasserabsorbierenden Polymerpartikel in ein 25 ml Becherglas eingewogen und gleichmäßig auf dessen Boden verteilt. Dann werden 20 ml einer 0,9 gew.-%igen Kochsalzlösung mittels eines Dispensers in ein zweites Becherglas dosiert und der Inhalt dieses Glases wird dem ersten zügig hinzugefügt und eine Stoppuhr gestartet. Sobald der letzte Tropfen Salzlösung absorbiert wird, was man am Verschwinden der Reflexion auf der Flüssigkeitsoberfläche erkennt, wird die Stoppuhr angehalten. Die genaue Flüssigkeitsmenge, die aus dem zweiten Becherglas ausgegossen und durch das Polymer im ersten Becherglas absorbiert wurde, wird durch Rückwägung des zweiten Becherglases genau bestimmt (=W2). Die für die Absorption benötigte Zeitspanne, die mit der Stoppuhr gemessen wurde, wird als t bezeichnet. Das Verschwinden des letzten Flüssigkeitstropfens auf der Oberfläche wird als Zeitpunkt t bestimmt.

Daraus errechnet sich die Anquellgeschwindigkeit (FSR) wie folgt:

$$FSR \ [g/g \ s] = W2/(W1xt)$$

Wenn der Feuchtegehalt der wasserabsorbierenden Polymerpartikel jedoch mehr als 3 Gew.-% beträgt, so ist das Gewicht W1 um diesen Feuchtegehalt zu korrigieren.

CIE-Farbzahl (L, a, b)

Die Farbmessung wird entsprechend dem CIELAB-Verfahren (Hunterlab, Band 8, Jahrgang 1996, Heft 7, Seiten 1 bis 4) mit einem Colorimeter, Modell "LabScan XE S/N LX17309" (HunterLab, Reston, US) durchgeführt. Dabei werden die Farben über die Koordinaten L, a und b eines dreidimensionalen Systems beschrieben. Dabei gibt L die Helligkeit an, wobei L = 0 schwarz und L = 100 weiß bedeutet. Die Werte für a und b geben die Position der Farbe auf den Farbachsen rot/grün bzw. gelb/blau an, wobei +a für rot, -a für grün, +b für gelb und -b für blau steht. Nach der Formel HC60 = L-3b wird der HC60-Wert berechnet.

Die Farbmessung entspricht dem Dreibereichsverfahren nach DIN 5033-6.

Die EDANA-Testmethoden sind beispielsweise erhältlich bei der EDANA, Avenue Eugene Plasky 157, B-1030 Brüssel, Belgien.

Beispiele

Herstellung des Basispolymers

Beispiel 1

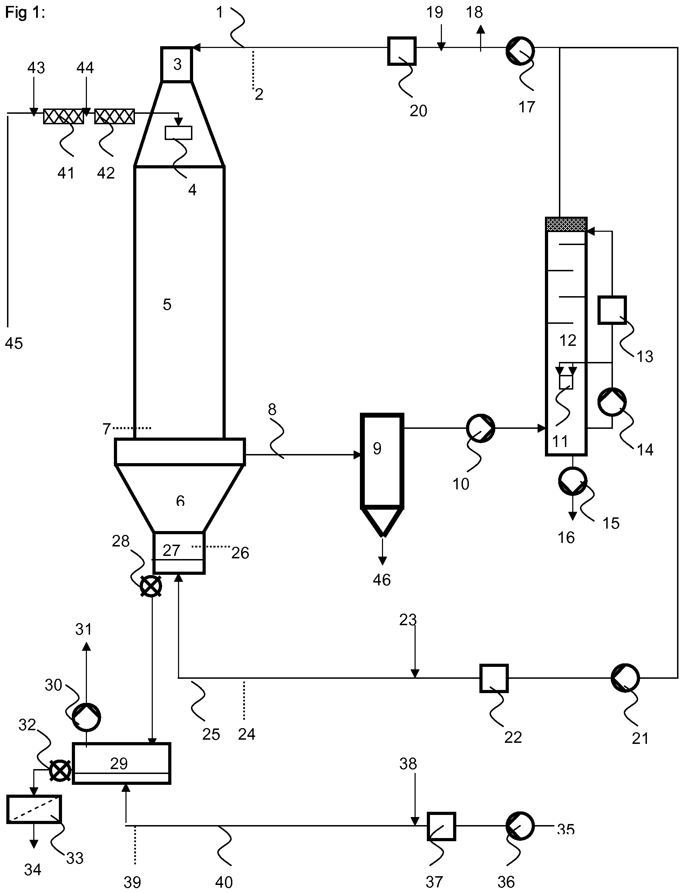

Das Verfahren wurde in einer Sprühtrocknungsanlage mit integriertem Wirbelbett (27) und einem externen Wirbelbett (29) durchgeführt, wie in Abbildung 1 von WO 2011/026876 A1 gezeigt. Der zylindrische Teil des Sprühtrockners (5) hatte eine Höhe von 22 m und einen Durchmesser von 3,4 m. Das interne Wirbelbett (IFB) hatte einen Durchmesser von 3 m und eine Wehrhöhe von 0,25 m. Die externe Wirbelbett (EFB) hatte eine Länge von 3,0 m, eine Breite von 0,65 m und eine Wehrhöhe von 0,5 m.

Das Trocknungsgas wurde über einen Gasverteiler (3) am oberen Ende des Sprühtrockners zugeführt. Das Trocknungsgas wurde teilweise über ein Gewebefilter (9) und eine Waschkolonne (12) zurückgeführt (Kreisgas). Als Trocknungsgas wurde Stickstoff mit einem Sauerstoffgehalt von 1 bis 4 Vol.-% eingesetzt. Vor Beginn der Polymerisation wurde die Anlage bis zu einem Sauerstoffgehalt von unter 4 Vol.-% mit Stickstoff gespült. Die Gasgeschwindigkeit des Trocknungsgases im zylindrischen Teil des Sprühtrockners (5) betrug 0,8 m/s. Der Druck innerhalb des Sprühtrockners war 4 mbar unterhalb des Umgebungsdruckes.

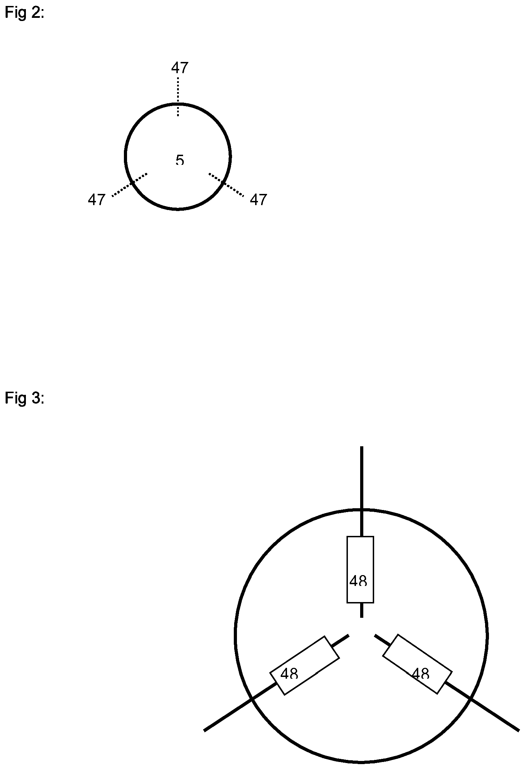

Die Ausgangstemperatur des Sprühtrockners wurde an drei Stellen am unteren Ende des zylindrischen Teils gemessen, wie in Abbildung 3 von WO 2011/026876 A1 gezeigt. Die drei Einzelmessungen (47) wurden zur Berechnung der mittleren Ausgangstemperatur verwendet. Das Kreisgas wurde aufgewärmt und die Dosierung von Monomerlösung wurde gestartet. Ab diesem Zeitpunkt wurde die mittlere Ausgangstemperatur durch Anpassung der Gaseingangstemperatur über den Wärmetauscher(20) auf 117°C geregelt.

Das Produkt wurde im internen Wirbelbett (27) bis zur Höhe des Wehrs gesammelt. Über die Leitung (25) wurde dem internen Wirbelbett (25) Trocknungsgas mit einer Temperatur von 122°C und einer relativen Feuchte von 4% zugeführt. Die Gasgeschwindigkeit im internen Wirbelbett (27) betrug 0,65 m/s. Die Verweilzeit des Produktes im internen Wirbelbett (27) betrug 120 Minuten.

Das Abgas des Sprühtrockners wurde über das Gewebefilter (9) der Waschkolonne (12) zugeführt. Der Flüssigkeitsstand innerhalb der Waschkolonne (12) wurde durch Abpumpen überschüssiger Flüssigkeit konstant gehalten. Die Flüssigkeit innerhalb der Waschkolonne (12) wurde mittels des Wärmetauschers (13) gekühlt und über die Düsen (11) im Gegenstrom gefördert, so dass die Temperatur innerhalb der Waschkolonne (12) auf 45°C geregelt wurde. Um Acrylsäure aus dem Abgas auszuwaschen wurde die Flüssigkeit in der Waschkolonne (12) durch Zusatz von Natronlauge alkalisch gestellt.

Das Abgas der Waschkolonne wurde auf die Leitungen (1) und (25) aufgeteilt. Die Temperaturen wurden mittels der Wärmetauscher (20) und (22) geregelt. Das erwärmte Trocknungsgas wurde dem Sprühtrockner über den Gasverteiler (3) zugeführt. Der Gasverteiler bestand aus einer Reihe von Platten und hatte je nach Gasmenge einen Druckverlust von 2 bis 4mbar

Das Produkt wurde aus dem internen Wirbelbett (27) mittels der Zellradschleuse (28) in das externe Wirbelbett (29) überführt. Über die Leitung (40) wurde dem externen Wirbelbett (29) Trocknungsgas mit einer Temperatur von 60°C zugeführt. Trocknungsgas war Luft. Die Gasgeschwindigkeit im externen Wirbelbett (29) betrug 0,8 m/s. Die Verweilzeit des Produktes im externen Wirbelbett (29) betrug 1 Minute.

Das Produkt wurde aus dem externen Wirbelbett (29) mittels der Zellradschleuse (32) auf das Sieb (33) gefördert. Mittels des Siebes (33) wurden Partikel mit einer Partikelgröße von größer 800 $\mu$m (Agglomerate) abgetrennt.

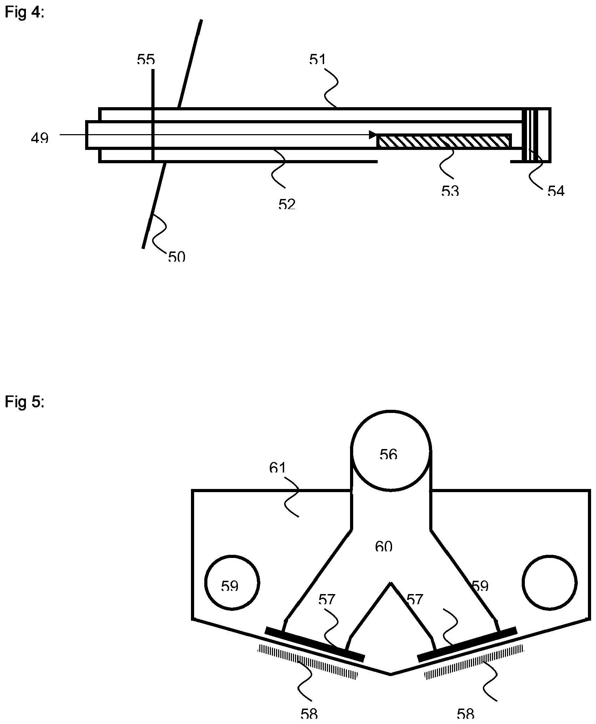

Zur Herstellung der Monomerlösung wurde zuerst frisch destillierte Acrylsäure mit dreifach ethoxyliertem Glyzerintriacrylat (Vernetzer) und anschließend mit 37,3gew.-%igem wässrigen Natriumacrylat versetzt. Durch Umpumpen über einen Wärmetauscher wurde die Temperatur der Monomerlösung auf 10°C gehalten. Im Umpumpkreis befand sich hinter der Pumpe ein Filter mit einer Maschenweite von 250 $\mu$m. Die Initiatoren wurden über die Leitungen (43) und (44) vor den statischen Mischern (41) und (42) der Monomerlösung zugesetzt. Natriumperoxodisulfat wurde mit einer Temperatur von 20°C über die Leitung (43) und eine Mischung von [2,2'-Azobis [2-(2-Imidazolin-2-yl)propan] dihydrochlorid und Brüggolit® FF7 (Brüggemann Chemicals; Heibronn; Deutschland) wurde mit einer Temperatur von 5°C über die Leitung (44) zugeführt. Jeder Initiator wurde im Kreis gepumpt und über Regelventile in jeder Vertropfereinheit dosiert. Hinter dem statischen Mischer (42) befand sich ein Filter mit einer Maschenweite von 140 $\mu$m. Zur Dosierung der Monomerlösung an der Spitze des Sprühtrockners wurden drei Vertropfereinheiten verwendet, wie in Abbildung 4 von WO 2011/026876 A1 gezeigt.

Eine Vertropfereinheit bestand aus einem äußeren Rohr (51) und einer Vertropferkassette (53), wie in Abbildung 5 von WO 2011/026876 A1gezeigt. Die Vertropferkassette (53) war mit einem inneren Rohr (52) verbunden. Das innere Rohr (52) hatte am Ende eine PTFE-Dichtung (54) und konnte während des Betriebs zu Wartungszwecken herausgezogen werden.

Die Temperatur der Vertropferkassette (61) wurde mittels Kühlwassers in den Kanälen (59) auf 8°C geregelt, wie in Abbildung 6 von WO 2011/026876 A1gezeigt. Der Vertropferkassette (61) hatte 256 Bohrungen mit einem Durchmesser von 170 $\mu$m und einem Abstand zwischen den Bohrungen von 15 mm. Die Vertropferkassette (61) hatte einen totraumfreien Strömungskanal (60) zur homogenen Verteilung der vorgemischten Monomerlösung und der Initiatorlösungen auf die beiden Vertropferplatten (57). Die beiden Vertropferplatten (57) hatten eine abgewinkelte Anordnung mit einem Winkel von 3°. Jede Vertropferplatte (57) war aus Edelstahl (Werkstoff Nr. 1.4571) und hatte eine Länge von 500 mm, eine Breite von 25 mm und eine Dicke von 1 mm.

Der Zulauf zum Sprühtrockner enthielt 10,45 Gew.-% Acrylsäure, 33,40 Gew.-% Natriumacrylat, 0,018 Gew.-% dreifach ethoxyliertes Glyzerintriacrylat (ca. 85gew.-%ig), 0,036 Gew.-% von [2,2'-Azobis[2-(2-Imidazolin-2-yl)propan] dihydrochlorid, 0,0029 Gew.-% Brüggolit® FF7 (Brüggemann Chemicals; Heibronn; Deutschland), 0,054 Gew.-% Natriumperoxodisulfat und Wasser. Brüggolit® FF7 wurde als 5gew.-%ige wässrige Lösung und Natriumperoxodisulfat wurde als 15gew.-%ige wässrige Lösung eingesetzt. Der Neutralisationsgrad betrug 71%. Der Zulauf pro Bohrung betrug 1,6 kg/h.

Die resultierenden Polymerpartikel hatten folgende Eigenschaften:

CRC von 32,6 g/g
AUL0.0psi von 37,5 g/g
AUL0.3psi von 24,2 g/g
AUL0.7psi von 12,0 g/g
Extrahierbare von 4,0 Gew.-%
Restmonomere 15.100 ppm
Feuchtegehalt von 6,1 Gew.-%
FSR von 0,28 g/gs
HC60 von 91,1
Farbwerte: L = 95,0 / a = 2,2 / b = 1,3

Die resultierenden Polymerpartikel hatten eine Schüttdichte von 0,74 g/ml und einen durchschnittlichen Partikeldurchmesser von 381$\mu$m.

Beispiel 2

Die Herstellung erfolgte analog zu Beispiel 1. Der Zulauf zum Sprühtrockner enthielt 10,45 Gew.-% Acrylsäure, 33,40 Gew.-% Natriumacrylat, 0,018 Gew.-% dreifach ethoxyliertes Glyzerintriacrylat (ca. 85gew.-%ig), 0,072 Gew.-% von [2,2'-Azobis[2-(2-Imidazolin-2-yl)propan]dihydrochlorid, 0,0029 Gew.-% Brüggolit® FF7 (Brüggemann Chemicals; Heibronn; Deutschland), 0,054 Gew.-% Natriumperoxodisulfat und Wasser. Brüggolit® FF7 wurde als 5gew.-%ige wässrige Lösung und Natriumperoxodisulfat wurde als 15gew.-%ige wässrige Lösung eingesetzt. Der Neutralisationsgrad betrug 71%. Der Zulauf pro Bohrung betrug 1,6 kg/h.

Die resultierenden Polymerpartikel hatten folgende Eigenschaften:

CRC von 47,0 g/g
AUL0.0psi von 55,1 g/g
AUL0.3psi von 25,1 g/g
AUL0.7psi von 7,6 g/g
Extrahierbare von 3,8 Gew.-%
Restmonomere 11.700 ppm
Feuchtegehalt von 6,1 Gew.-%
FSR von 0,14 g/gs
HC60 von 92,5
Farbwerte: L = 96,1 / a = 1,7 / b = 1,2

Die resultierenden Polymerpartikel hatten eine Schüttdichte von 0,75 g/ml und einen durchschnittlichen Partikeldurchmesser von 385μm

Thermische Nachbehandlung

Vergleichsbespiele VB1 und 2 und Beispiele 3 bis 12

1,5 kg wasserabsorbierende Polymerpartikel aus Beispiel 1 wurden in einem Pflugscharmischer vom Typ M5R (Gebr. Lödige Maschinenbau GmbH; Paderborn; Deutschland) thermisch nachbehandelt.

Hierzu wurde der Pflugscharmischer eine Stunde auf eine Wandtemperatur von 100°C vorgeheizt. Nach dem Aufheizen wurden die wasserabsorbierenden Polymerpartikel aus Beispiel 1 in den Pflugscharmischer eingetragen. Anschließend wurde der Motor gestartet und die Drehzahl auf 60 U/min eingestellt. Nach einer Aufheizzeit von 20 Minuten wurde die auf ca. 80°C erwärmte erste Hälfte der Sprühsubstanz gemäß Tabelle 1 mittels einer Büchi Zweistoffdüse mit 1 bar Stickstoff innerhalb von 6,5 Minuten von oben auf das Produkt aufgesprüht. Anschließend wurde 5 Minuten weiter gerührt und dann die zweite Hälfte der Sprühsubstanz unter analogen Bedingungen aufgesprüht. Das Produkt wurde bei einer Temperatur von 80°C für 30 Minuten nachgerührt. Hierbei wurde ein Stickstoffstrom von 60 l/h durch den Pflugscharmischer geleitet. Das abgekühlte Produkt wurde auf einer Plansiebmaschine vom Typ AS400 (Retsch GmbH; Haan; Deutschland) auf <850 μm abgesiebt.

Vergleichsbeispiel VB3 und Beispiele 13 bis 15

45 kg wasserabsorbierende Polymerpartikel aus Beispiel 2 wurden im Pflugscharmischer vom Typ FM130 (Gebr. Lödige Maschinenbau GmbH; Paderborn; Deutschland) thermisch nachbehandelt. Während des gesamten Versuches betrug die Drehzahl des Pflugscharmischers 60 U/min und die Leistungsaufnahme 6,4 bis 6,5 W. Der Mischraum wurde mit ca. 10 Nm$^3$/h Stickstoff, bei ca. 3 bar absolutem Vordruck und einer Gastemperatur von 180 °C durchströmt. Die Froude-Zahl betrug 0.93.

Vor dem Befüllen des Mischraumes wurde der Pflugscharmischer auf eine Manteltemperatur von 150°C vorgewärmt. Anschließend wurden die wasserabsorbierenden Polymerpartikel aus Beispiel 2 in den Pflugscharmischer eingetragen und 20 Minuten unter Rühren erwärmt. Die Produkttemperatur stieg auf 70°C.

Im nächsten Schritt wurde der Stickstoff mit einer Dampfmenge von ca. 6,5 kg/h beaufschlagt. Die Dampfmenge wurde dabei so geregelt, dass sich eine relative Gasfeuchte von 30% einstellte. Die Gasfeuchte wurde mittels eines Feuchtemessers vom Typ HygroFlex (Rotronic Messgeräte GmbH; Nürnberg; Deutschland) bestimmt. Die Gastemperatur betrug 180 °C. Während dieser Zeit wurden gemäß Tabelle 2 in 4 gleichmäßigen Portionen Wasser mit einer Temperatur von 25°C mittels einer Zweistoffdüse vom Typ 970S4 1,2mm Düsen-Schlick GmbH; Untersiemau; Deutschland in je 8 Minuten aufgesprüht. Die Zweistoffdüse wurde mit Stickstoff (3 bar) betrieben.

Am Ende des Sprühvorganges hatte das Produkt eine Temperatur von ca. 85°C. Die Dampfbeladung und die Stickstofferwärmung wurden abgestellt. Das Produkt wurde nun für insgesamt 15 Minuten unter Rühren nachgetrocknet.

Kurz vor dem Entleeren hatte das Produkt eine Temperatur von ca. 76°C.

Nach dem Versuchsende wurde das abgekühlte Produkt geteilt und auf einer Plansiebmaschine vom Typ AS400 (Retsch GmbH; Haan; Deutschland) auf <850 μm abgesiebt.

Tabelle 1

| Bsp. | Sprühsubstanz | Partikel >850 μm [Gew.-%] | Agglomerate [Gew.-%] | Feuchtegehalt [Gew.-%] | Restmonomere [ppm] |
|---|---|---|---|---|---|
| 1 | - | 3,1 | 0 | 6,1 | 16000 |
| VB1 | 225 g Wasser | 30,4 | 27,3 | 10,7 | 2400 |
| VB2 | 225g Wasser + 0,15 g Span20 | 22,4 | 19,3 | 11,9 | 1700 |
| 3 | 225g Wasser + 0,15 g Plantacare 818UP | 5,2 | 2,1 | 12,3 | 1100 |
| 4 | 225g Wasser + 0,15 g Plantacare 1200UP | 8,9 | 5,8 | 12,3 | 1100 |
| 5 | 225g Wasser + 0,15 g Plantacare K55 | 12,9 | 9,8 | 14,3 | 1800 |
| 6 | 225g Wasser + 0,15 g Euperlan PCO | 10,8 | 7,7 | 11,5 | 1100 |
| 7 | 225g Wasser + 0,15 g Lutensol AO7 | 13,8 | 10,7 | 9,3 | 2800 |
| 8 | 225g Wasser + 0,15 g Lutensol XL70 | 13,9 | 10,8 | 9,3 | 3400 |
| 9 | 225g Wasser + 0,15 g Lutensol FA12K | 14,7 | 11,6 | 10,1 | 2200 |
| 10 | 225g Wasser + 0,15 g Arquad 2HT-75 | 5,7 | 2,6 | 9,8 | 2600 |
| 11 | 225g Wasser + 0,15 g Emulan EL40 | 12,8 | 9,7 | 9,8 | 2500 |
| 12 | 225g Wasser + 0,15 g Luviquat Mono CP | 2,6 | 0,0 | 12,5 | 2000 |

Tabelle 2

| Bsp. | Sprühsubstanz | Partikel >850 $\mu$m [Gew.-%] | Agglomerate [Gew.-%] | Feuchtegehalt [Gew.-%] | Restmonomere [ppm] | Oberflächenspannung [mN/m] |
|---|---|---|---|---|---|---|
| 2 | | 3,3 | | 6,3 | 11680 | 72,0 |
| VB3 | 4 x (843g Wasser) | 31,3 | 28,8 | 9,7 | 2100 | 71,8 |
| 13 | 4 x (843g Wasser + 1,125 g Plantacare 818UP) | 4,2 | 0,6 | 8,9 | 1200 | 72,1 |
| 14 | 4 x (843g Wasser + 2,25 g Plantacare 818UP) | 2,1 | 0,0 | 9,3 | 900 | 70,9 |
| 15 | 4 x (843g Wasser + 3,375 g Luviquat Mono CP | 3,5 | 0,2 | 8,8 | 1000 | 64,7 |

**EP 2 950 829 B1**

Als Tenside sind folgende Verbindungen verwendet worden

Span®20 (Sorbitanmonooctoat, Croda International Plc, CAS-Nr. CAS 1338-39-2)

Plantacare®818UP (ca. 52 gew.-%ige wässrige Lösung von Cocoglycosid, BASF SE, CAS-Nr.110615-47-9 / 68515-73-1)

Plantacare®1200UP (ca. 50 gew.-%ige wässrige Lösung von Laurylglycosid, BASF SE, CAS-Nr. CAS-Nr.110615-47-9)

Plantacare®K55 (ca. 47 gew.-%ige wässrige Lösung von einer Mischung aus Laurylglycosid und Cocomidpropylbetain, BASF SE, CAS-Nr. CAS-Nr.110615-47-9/61789-40-0)

Plantacare®PS10 (ca. 60 gew.-%ige wässrige Lösung von einer Mischung aus Natriumlaurethersulfat und Laurylglycosid, BASF SE, CAS-Nr.110615-47-9 / 68891-38-3)

Euperlan®PCO (ca. 43 gew.-%ige Dispersion von einer Mischung aus Stryrol/Acrylat-Copolmer mit Cocoglycosid, BASF SE)

Lutensol®AO7 (ethoxlyierter Oxoalkohol, BASF SE): Lutensol®AO7 ist ein nichtionisches flüssiges Tensid auf der Basis eines gesättigten $C_{13}$/$C_{15}$-Oxoalkohols, der aus ca. 67% $C_{13}$-Oxoalkohol und ca. 33% $C_{15}$-Oxoalkohol besteht und überwiegend unverzweigt ist. Die Zusammensetzung von Lutensol®AO7 wird durch folgende allgemeine Struk-turformel wiedergegeben: $RO(CH_2CH_2O)_7H$ mit R = $C_{13}$/$C_{15}$-Oxoalkohol. Lutensol®AO7 hat eine Viskosität von ca. 100 mPas, einen HLB-Wert von ca. 12 und einen Aktivgehalt von 100%.

Lutensol®XL70 (ethoxylierter Guerbetalkohol, BASF SE): Lutensol®XL70 ist ein nichtionisches flüssiges Tensid auf der Basis eines $C_{10}$-Guerbet Alkohols. Die Zusammensetzung von Lutensol®XL70 wird durch folgende allge-meine Strukturformel wiedergegeben: $RO(CH_2CH_2O)_7H$ mit R = $C_{10}$-Guerbet Alkohol. Lutensol®XL70 hat eine Viskosität von ca. 70 mPas, einen HLB-Wert von ca. 13 und einen Aktivgehalt von 100%.

Lutensol®FA12K (ethoxyliertes Kokosalkylamin, BASF SE): Lutensol®FA12K ist ein nichtionisches flüssiges Tensid auf der Basis von Kokosfettamin. Die Zusammensetzung von Lutensol®FA12K wird durch folgende allgemeine Strukturformel wiedergegeben: $R-N[(CH_2CH_2O)_6H]_2$ mit $R-NH_2$ = Kokosfettamin. Lutensol®FA12K hat eine Viskosität von ca. 190 mPas und einen Aktivgehalt von 100%.

Arquad®2HT-75 (Ditallowdimethylammoniumchlorid, AkzoNobel GmbH, CAS-Nr. 61789-80-8)

Emulan®EL40 (ethoxyliertes Rizinusöl, BASF SE): Emulan®EL40 ist ein nichtionisches flüssiges Tensid auf der Basis von Rizinusöl. Die Zusammensetzung der Emulan®EL40 wird durch folgende allgemeine Strukturformel wiedergegeben: $ROCH_2CH(OR)CH_2OR$ mit R = $H_3C(CH_2)_5CH[O(CH_2CH_2O)_{40/3}H]CH_2CH=CH(CH_2)_7COO-$. Emu-lan®EL40 hat eine Viskosität von ca. 3000 mPas, einen HLB-Wert von ca. 12 und einen Aktivgehalt von 100%.

Luviquat®Mono CP (ca. 30 gew.-%ige wässrige Lösung von Hexadecyl-(2-hydroxyethyl)dimethylammonium-dihydrogenphosphat, BASF SE, CAS-Nr. 85563-48-0)

**Patentansprüche**

1. Verfahren zur Entfernung von Restmonomeren aus wasserabsorbierenden Polymerpartikeln, wobei die wasserabsorbierenden Polymerpartikel in einem Mischer mit rotierenden Mischwerkzeugen thermisch nachbehandelt werden, die wasserabsorbierenden Polymerpartikel während der thermischen Nachbehandlung eine Temperatur von mindestens 60°C und einen Wassergehalt von mindestens 8 Gew.-% aufweisen, wobei vor oder während der thermischen Nachbehandlung Wasser oder eine wässrige Lösung und/oder während der thermischen Nachbehandlung ein Wasserdampf enthaltender Gasstrom zugesetzt wird, **dadurch gekennzeichnet, dass** die thermische Nachbehandlung in Gegenwart mindestens eines Tensids durchgeführt wird, das mindestens eine Tensid eine polare und eine unpolare Gruppe aufweist, die polare und die unpolare Gruppe des Tensids nicht über eine Carbonsäureester-Gruppe verbunden sind, die polare Gruppe mindestens eine Hydroxyl-Gruppe, eine kationische Gruppe oder eine anionische Gruppe aufweist und die unpolare Gruppe eine $C_4$- bis $C_{20}$-Alkylkette aufweist.

**2.** Verfahren gemäß Anspruch 1, **dadurch gekennzeichnet, dass** die polare Gruppe eine kationische Gruppe ist.

**3.** Verfahren gemäß Anspruch 1, **dadurch gekennzeichnet, dass** das Tensid eine Tensidmischung ist.

**4.** Verfahren gemäß Anspruch 3, **dadurch gekennzeichnet, dass** die Tenside der Tensidmischung unterschiedliche lineare und/oder verzweigte Alkylketten als unpolare Gruppen enthalten.

**5.** Verfahren gemäß einem der Ansprüche 1 bis 4, **dadurch gekennzeichnet, dass** von 0,0005 bis 0,01 Gew.-% des Tensids, bezogen auf die wasserabsorbierenden Polymerpartikel, eingesetzt wird.

**6.** Verfahren gemäß einem der Ansprüche 1 bis 5, **dadurch gekennzeichnet, dass** die wasserabsorbierenden Polymerpartikel während der thermischen Nachbehandlung eine Temperatur von 80 bis 110°C aufweisen.

**7.** Verfahren gemäß einem der Ansprüche 1 bis 6, **dadurch gekennzeichnet, dass** vor oder während der thermischen Nachbehandlung, bezogen auf die wasserabsorbierenden Polymerpartikel, von 0,05 bis 20 Gew.-% Wasser oder einer wässrigen Lösung zugesetzt wird.

**8.** Verfahren gemäß einem der Ansprüche 1 bis 7, **dadurch gekennzeichnet, dass** der während der thermischen Nachbehandlung zugesetzte Gasstrom von 0,05 bis 1,0 kg Wasserdampf pro kg trockenes Gas enthält.

**9.** Verfahren gemäß einem der Ansprüche 1 bis 8, **dadurch gekennzeichnet, dass** die mittlere Verweilzeit im Mischer von 10 bis 120 Minuten beträgt.

**10.** Verfahren gemäß einem der Ansprüche 1 bis 9, **dadurch gekennzeichnet, dass** das zur thermischen Nachbehandlung verwendete Gasvolumen in einem diskontinuierlichen Mischer von 0,01 bis 5 Nm$^3$/h pro kg wasserabsorbierende Polymerpartikel oder in einem kontinuierlichen Mischer von 0,01 bis 5 Nm$^3$/h pro kg/h durchgesetzter wasserabsorbierender Polymerpartikel beträgt.

**11.** Verfahren gemäß einem der Ansprüche 1 bis 10, **dadurch gekennzeichnet, dass** die thermische Nachbehandlung in einem Horizontalmischer durchgeführt wird.

**12.** Verfahren gemäß einem der Ansprüche 1 bis 11, **dadurch gekennzeichnet, dass** die wasserabsorbierenden Polymerpartikel durch Polymerisation einer Monomerlösung, enthaltend

a) mindestens ein ethylenisch ungesättigtes, säuregruppentragendes Monomer, das zumindest teilweise neutralisiert sein kann,
b) mindestens einen Vernetzer,
c) mindestens einen Initiator
d) optional ein oder mehrere mit den unter a) genannten Monomeren copolymerisierbare ethylenisch ungesättigte Monomere,
e) optional ein oder mehrere wasserlösliche Polymere oder Copolymere und
f) Wasser,

erhalten werden.

**13.** Verfahren gemäß Anspruch 12, **dadurch gekennzeichnet, dass** das Monomer a) zu mindestens 25 mol-% neutralisiert ist.

**14.** Verfahren gemäß Anspruch 12 oder 13, **dadurch gekennzeichnet, dass** das Monomer a) zu mindestens 50 mol-% Acrylsäure ist

**15.** Verfahren gemäß einem der Ansprüche 12 bis 14, **dadurch gekennzeichnet, dass** die Monomerlösung mindestens 0,1 Gew.-% Vernetzer b), bezogen auf unneutralisiertes Monomer a) enthält.

**Claims**

**1.** A process for removing residual monomers from water-absorbing polymer particles by thermally aftertreating the water-absorbing polymer particles in a mixer with rotating mixing tools, the water-absorbing polymer particles during the thermal aftertreatment having a temperature of at least 60°C and a water content of at least 8% by weight, with addition of water or an aqueous solution before or during the thermal aftertreatment and/or of a steam-comprising gas stream during the thermal aftertreatment, which comprises performing the thermal aftertreatment in the presence of at least one surfactant, the at least one surfactant having a polar group and a nonpolar group, the polar group and the nonpolar group of the surfactant not being joined via a carboxylic ester group, and the polar group having at least one hydroxyl group, a cationic group or an anionic group and the nonpolar group having a $C_4$- to $C_{20}$-alkyl chain.

2. The process according to claim 1, wherein the polar group is a cationic group.

3. The process according to claim 1, wherein the surfactant is a surfactant mixture.

4. The process according to claim 3, wherein the surfactants in the surfactant mixture comprise different linear and/or branched alkyl chains as nonpolar groups.

5. The process according to any of claims 1 to 4, wherein from 0.0005 to 0.01% by weight of the surfactant, based on the water-absorbing polymer particles, is used.

6. The process according to any of claims 1 to 5, wherein the water-absorbing polymer particles have a temperature of 80 to 110°C during the thermal aftertreatment.

7. The process according to any of claims 1 to 6, wherein from 0.05 to 20% by weight of water or an aqueous solution is added before or during the thermal aftertreatment, based on the water-absorbing polymer particles.

8. The process according to any of claims 1 to 7, wherein the gas stream added during the thermal aftertreatment comprises from 0.05 to 1.0 kg of steam per kg of dry gas.

9. The process according to any of claims 1 to 8, wherein the mean residence time in the mixer is from 10 to 120 minutes.

10. The process according to any of claims 1 to 9, wherein the gas volume used for thermal aftertreatment in a batchwise mixer is from 0.01 to 5 $m^3$ (STP)/h per kg of water-absorbing polymer particles, or, in a continuous mixer, from 0.01 to 5 $m^3$ (STP)/h per kg/h of water-absorbing polymer particle throughput.

11. The process according to any of claims 1 to 10, wherein the thermal aftertreatment is performed in a horizontal mixer.

12. The process according to any of claims 1 to 11, wherein the water-absorbing polymer particles are obtained by polymerizing a monomer solution comprising

    a) at least one ethylenically unsaturated monomer which bears acid groups and may be at least partly neutralized,
    b) at least one crosslinker,
    c) at least one initiator,
    d) optionally one or more ethylenically unsaturated monomers copolymerizable with the monomers mentioned under a),
    e) optionally one or more water-soluble polymers or copolymers and
    f) water.

13. The process according to claim 12, wherein the monomer a) has been neutralized to an extent of at least 25 mol%.

14. The process according to claim 12 or 13, wherein the monomer a) is acrylic acid to an extent of at least 50 mol%.

15. The process according to any of claims 12 to 14, wherein the monomer solution comprises at least 0.1% by weight of crosslinker b), based on unneutralized monomer a).

**Revendications**

1. Procédé pour éliminer les monomères résiduels de particules polymères absorbant l'eau, les particules polymères absorbant l'eau étant post-traitées thermiquement dans un mélangeur présentant des outils de mélange rotatifs, les particules polymères absorbant l'eau présentant, pendant le post-traitement thermique, une température d'au moins 60°C et une teneur en eau d'au moins 8% en poids, de l'eau ou une solution aqueuse étant ajoutée avant ou pendant le post-traitement thermique et/ou un flux gazeux contenant de la vapeur d'eau étant ajouté pendant le post-traitement thermique, **caractérisé en ce que** le post-traitement thermique est réalisé en présence d'au moins un tensioactif, ledit au moins un tensioactif présente un groupe polaire et un groupe non polaire, le groupe polaire et le groupe non polaire du tensioactif ne sont pas reliés via un groupe ester d'acide carboxylique, le groupe polaire présente au moins un groupe hydroxyle, un groupe cationique ou un groupe anionique et le groupe non polaire présente une chaîne alkyle en $C_4$-$C_{20}$.

2. Procédé selon la revendication 1, **caractérisé en ce que** le groupe polaire est un groupe cationique.

3. Procédé selon la revendication 1, **caractérisé en ce que** le tensioactif est un mélange de tensioactifs.

4. Procédé selon la revendication 3, **caractérisé en ce que** les tensioactifs du mélange de tensioactifs contiennent des chaînes alkyle linéaires et/ou ramifiées différentes en tant que groupes non polaires.

5. Procédé selon l'une quelconque des revendications 1 à 4, **caractérisé en ce que** 0,0005 à 0,01% en poids, par rapport aux particules polymères absorbant l'eau, de tensioactif est utilisé.

6. Procédé selon l'une quelconque des revendications 1 à 5, **caractérisé en ce que** les particules polymères absorbant l'eau présentent, pendant le post-traitement thermique, une température de 80 à 110°C.

7. Procédé selon l'une quelconque des revendications 1 à 6, **caractérisé en ce que** 0,05 à 20% en poids, par rapport aux particules polymères absorbant l'eau, d'eau ou d'une solution aqueuse sont ajoutés avant ou pendant le post-traitement thermique.

8. Procédé selon l'une quelconque des revendications 1 à 7, **caractérisé en ce que** le flux gazeux ajouté pendant le post-traitement thermique contient 0,05 à 1,0 kg de vapeur d'eau par kg de gaz sec.

9. Procédé selon l'une quelconque des revendications 1 à 8, **caractérisé en ce que** le temps de séjour moyen dans le mélangeur est de 10 à 120 minutes.

10. Procédé selon l'une quelconque des revendications 1 à 9, **caractérisé en ce que** le volume de gaz utilisé pour le post-traitement thermique, dans un mélangeur discontinu, est de 0,01 à 5 $Nm^3$/h par kg de particules polymères absorbant l'eau ou, dans un mélangeur continu, de 0,01 à 5 $Nm^3$/h par kg/h de particules polymères absorbant l'eau qui passent.

11. Procédé selon l'une quelconque des revendications 1 à 10, **caractérisé en ce que** le post-traitement thermique est réalisé dans un mélangeur horizontal.

12. Procédé selon l'une quelconque des revendications 1 à 11, **caractérisé en ce que** les particules polymères absorbant l'eau sont obtenues par polymérisation d'une solution de monomères, contenant

   a) au moins un monomère éthyléniquement insaturé, portant des groupes acides, qui peut être au moins partiellement neutralisé,
   b) au moins un réticulant,
   c) au moins un initiateur,
   d) éventuellement un ou plusieurs monomères éthyléniquement insaturés copolymérisables avec les monomères cités en a),
   e) éventuellement un ou plusieurs polymères ou copolymères solubles dans l'eau et
   f) de l'eau.

13. Procédé selon la revendication 12, **caractérisé en ce que** le monomère a) est neutralisé à raison d'au moins 25% en mole.

14. Procédé selon la revendication 12 ou 13, **caractérisé en ce que** le monomère a) est, à raison d'au moins 50% en mole, de l'acide acrylique.

**15.** Procédé selon l'une quelconque des revendications 12 à 14, **caractérisé en ce que** la solution de monomères contient au moins 0,1% en poids de réticulant b), par rapport au monomère a) non neutralisé.

Fig 1:

Fig 2:

Fig 3:

Fig 4:

Fig 5:

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008095901 A1
- WO 2011117215 A1
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2
- WO 2001038402 A1
- DE 3825366 A1
- US 6241928 B

- WO 2008052971 A1
- WO 2011026876 A1
- US 3243321 A
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- DE 3713601 A1
- WO 2012045705 A1

**In der Beschreibung aufgeführte Nicht-Patentliteratur**

- **F.L. BUCHHOLZ ; A.T. GRAHAM.** Modern Superabsorbent Polymer Technology. Wiley-VCH, 1998, 71-103
- *CHEMICAL ABSTRACTS,* 110615-47-9/68515-73-1
- *CHEMICAL ABSTRACTS,* 110615-47-9
- *CHEMICAL ABSTRACTS,* 110615-47-9/61789-40-0
- *CHEMICAL ABSTRACTS,* 110615-47-9/68891-38-3
- *CHEMICAL ABSTRACTS,* 110615-47-9/1337-33-4
- **VON S.MONTGOMERY ; D.KENNEDY ; N.O'DOWD.** PVD and CVD Coating for the Metal Forming Industry. *Conference Paper,* 2010

- Ullmann's Encyclopedia of Industrial Chemistry
- **F.L. BUCHHOLZ ; AT. GRAHAM.** Modern Superabsorbent Polymer Technology. Wiley-VCH, 1998, 252-258
- *Hunterlab,* 1996, vol. 8 (7), 1-4
- *CHEMICAL ABSTRACTS,* 1338-39-2
- *CHEMICAL ABSTRACTS,* 110615-47-9 / 68515-73-1
- *CHEMICAL ABSTRACTS,* 110615-47-9 / 68891-38-3
- *CHEMICAL ABSTRACTS,* 61789-80-8
- *CHEMICAL ABSTRACTS,* 85563-48-0